# EUROPEAN PATENT APPLICATION

(11) **EP 2 289 896 A1**
(43) Date of publication of application: **02.03.2011**
(21) Application number: 09168288.0
(22) Date of filing: 20.08.2009
(51) Int. Cl.: C07D 493/10

(54) **Process for the manufacture of 3-dibutylamino-6-methyl-7-anilinofluoran**

(71) Applicant: BASF SE, 67056 Ludwigshafen (DE)
(72) Inventor: The designation of the inventor has not yet been filed
(74) Representative: Bernhardt, Wolfgang Willy-Hans

(57) **Abstract**

A process for the preparation of 3-dibutylamino-6-methyl-7-anilinofluoran and a heating- and pressure sensitive recording material are claimed.

## Description

The present invention relates to a process for the manufacture of 3-dibutylamino-6-methyl-7-anilinofluoran. Manufacturing processes for 3-dibutylamino-6-methyl-7-anilinofluoran are known e.g. from US 5,166,350. US 5,166,350 describes a process in which in a first step a ketonic acid is reacted with a substituted phenol derivative in concentrated or fuming sulphuric acid or a mixture thereof. In the second step the reaction mixture of the first step is added to an aqueous mixture containing a non-polar solvent and a base. In the third step the organic phase is separated, and then the fluoran product is obtained by removing the organic solvent from this organic phase.

Any coloured impurities in the colour former can lead to a reduction in the background whiteness of the heat-sensitive recording material. Likewise, in pressure-sensitive recording applications, the presence of coloured impurities in the colour former can lead to coloration of the microencapsulated colour former solution which, in turn, can lead to a coloration of the resulting CB sheet.

It has now been found that not only a white colored 3-dibutylamino-6-methyl-7-anilinofluoran can be obtained, but also the used sulphuric acid can be recovered and reused, less organic by-products are observed, and the background whiteness is improved.

Accordingly, the instant invention concerns a process for the preparation of 3-dibutylamino-6-methyl-7-anilinofluoran which process comprises reacting 4-methoxy-2-methyldiphenylamine with 2-(4'-di-n-butylamino-2'-hydroxybenzoyl)benzoic acid in fuming sulphuric acid or a mixture thereof with sulphuric acid, treating the reaction mass with water, a non-polar solvent and a base, and isolating the final product, wherein the following steps are carried out:
(i) carrying out the reaction between 4-methoxy-2-methyldiphenylamine and 2-(4'-di-n-butylamino-2'-hydroxybenzoyl)benzoic acid in fuming sulphuric acid or a mixture thereof with sulphuric acid at a temperature below 30°C,
(ii) then adding water to the reaction mass,
(iii) afterwards adding a non-polar solvent to the diluted reaction mass,
(iv) followed by separating the obtained organic phase,
(v) thereafter treating the organic phase with an aqueous base, and then heating at a temperature of 50° to 90°C, and finally
(vi) isolating 3-dibutylamino-6-methyl-7-anilinofluoran.

Obviously, the addition of a base is done after the separation of the sulphuric acid containing aqueous phase in contrast to the process of US 5,166,350.

In step (i) 4-methoxy-2-methyldiphenylamine and 2-(4'-di-n-butylamino-2'-hydroxybenzoyl)benzoic acid are preferably used in molar amounts, in a more preferred embodiment of this invention the molar ratio is chosen in the range of 0.90:1 to 1.10:1, preferably 0.95:1 to 1.05:1 (4-methoxy-2-methyldiphenylamine : 2-(4'-di-n-butylamino-2'-hydroxybenzoyl)benzoic acid).

Usually, oleum or a mixture of oleum and sulphuric acid is used, wherein the sulphuric acid has a sulphuric acid content in the range of from 50 to 100% by weigth, preferably 90 to 98% b.w.

The SO₃ content of the chosen oleum is preferably in the range of 20 to 22 weight%.

As a rule, oleum or the mixture of oleum and sulphuric acid is used in an weight range of 2:1 to 5:1 based on 2-(4'-di-n-butylamino-2'-hydroxybenzoyl)benzoic acid.

The reaction is carried out at a temperature below 30°C, preferably in a range of 10 to 29°C, more preferably between 10 and 20°C.

In a preferred embodiment 2'-carboxy-4-dibutylamino-2-hydroxybenzophenone is added to a mixture of oleum (containing 20% SO₃) and sulfuric acid (98% b.w.), which has been cooled to a temperature in the range of from 15 to 25°C before the addition. Preferably the addition of 2'-carboxy-4-dibutylamino-2-hydroxybenzophenone is carried out during a time period between 1 hour and 10 hours while usually maintaining the temperature at or below 20°C, i.e. in a range of from 10 to 20°C.

In a further preferred embodiment the above mixture of 2'-carboxy-4-dibutylamino-2-hydroxybenzophenone/oleum and sulfuric acid is kept at a temperature in the range of from 10 to 20°C for further 30 to 60 minutes before 4-methoxy-2-methyldiphenylamine is added preferably in portions over a time period in the range of from 1 to 10 hours, preferably from 4 to 6 hours, while, as a rule, the temperature is maintained between 10 to 20°C.

In step (ii) water is added to the reaction mass. In general, the amount of water to benzophenone is choosen in the range of from 10:1 to1:1 (weight ratios).

In a preferred embodiment, the water is added in more than one step, preferably in two steps. In a more preferred embodiment, the temperature of the water is kept in a range of from 15 to 25°C. In the most preferred embodiment, a quarter of the water amount is added during a time period of 1 to 5 hours while the reaction mixture is cooled externally in order to keep the temperature in a range of from 30 to 70°C. Then the rest of the water is added over 30 mins, maintaining the reaction mass temperature in a range of from 30 to 70°C.

In step (iii) a non-polar solvent is added to the diluted reaction mass. As non-polar solvents e.g. toluene, o-, m-, p-xylene, monochlorobenzene, dichlorobenzene, trichlorobenzene, nitrobenzene or mixtures thereof can be used, preferably toluene.

Usually, the weight ratio of non-polar solvent to benzophenone is chosen in the range of from 5:1 to 1:1, preferably from 2:1 to 1.5:1.

Generally, the temperature during the addition of the non-polar solvent is chosen in the range of from 60 to 80°C, preferably from 65 to 70°C. Preferably, the reaction mass is stirred during the addition.

In a preferred embodiment, the thus obtained mixture is allowed to settle for 30 to 240 minutes, and most preferred the temperature is kept during this time in a range of from 50 to 70°C.

In step (iv) the organic phase is separated from the aqueous phase. This separation can be carried out by all well known means such as decantation or removal of the lower layer in an appropriate device, e.g. a reaction vessel having a nozzle at the bottom.

In a preferred embodiment the lower aqueous layer is removed and afterwards a water-soluble polar solvent such as a C₁-C₃-alkanol, e.g. methanol, ethanol, n- or i-propanol, preferably methanol, is added to remaining organic phase. Usually, the organic phase is stirred at a temperature in the range of from 50°C to the boiling point of the water-soluble alkanol, e.g. 65°C if methanol is used, for a desired period of time, e.g. 15 to 60 minutes.

In step (v) an aqueous base is added to the organic phase and the reaction mixture is then heated to a temperature in the range of from 50 to 90°C, preferably 70 to 85°C.

Suitable bases are alkali metal hydroxides such as sodium hydroxide, potassium hydroxide, ammonia, alkali metal carbonates such as sodium carbonate or potassium carbonate, alkali metal bicarbonates (or hydrogencarbonates) such as sodium hydrogencarbonate or potassium hydrogencarbonate, ammonium carbonate or ammonium bicarbonate, di-C₁-C₄-alkyl- or tri-C₁-C₄-alkylamines such as diethylamine or triethylamine, or di-C₁-C₄- alkanol- or tri-C₁-C₄-alkanolamines such as diethanolamine, diisopropanolamine, triethanolamine and triisopropanolamine ; and mixtures thereof. Most preferred is sodium hydroxide.

Usually, the molar ratio of the base to 2'-carboxy-4-dibutylamino-2-hydroxybenzophenone is chosen in the range of from 1:1 to 20:1, preferably from 10:1 to 15:1.

According to present observations the time period for the heat treatment is not critical. However, as a rule, the heat treatment it is carried out during a time period in the range of 30 minutes to 4 hours, preferably from 2 to 3 hours.

Generally, the base is added in the form of an aqueous solution.

In a more preferred embodiment, an alkali metal hydroxide solution as is used. Advantageously the concentration of the alkali metal hydroxide in this solution is in the range of from 10 to 50% by weight, preferably from 25 to 40% b.w.

In an even more preferred embodiment a mixture of an alkali metal hydroxide solution and a non-polar solvent such as one mentioned above or a mixture therefrom, e.g. toluene, is used. Usually the weight ratio of water / alkali metal hydroxide is chosen in the range of from 10:1 1 to 1:1, preferably from 5:1 to 1.5:1, and the weight ratio of non-polar solvent to alkali metal hydroxide is chosen in the range of from 5:1 to 1:1, preferably from 3:1 to 2:1.

Preferably, this mixture is heated to a temperature in the range of from 40 to 80°C before the reaction mixture from step (iv) is added.

In a particularly preferred embodiment a mixture of toluene, water and sodium hydroxide having a temperature in the range of from 60 to 80°C is used, wherein the weight ratio of water to sodium hydroxide is chosen in the range of from 5:1 to 1.5:1, and the weight ratio of toluene to sodium hydroxide is chosen in the range of from 3:1 to 2:1. To this mixture the reaction mixture of step (iv) is added during a time period in the range of from 30 minutes to 4 hours.

If desired the yield can be increased in washing out the reaction vessel of step (iv) with a polar solvent such as a C₁-C₃-alkanol as mentioned above, preferably methanol. As a rule, the polar solvent is used in a heated form, e.g. heated up to a temperature of 5 to 20°C below its boiling point. The amount of the polar solvent depends on different circumstances such as the used vessel, and is not critical up to observations to date. However, e.g. if methanol is used, the weight ratio of toluene used in step (iii) and methanol can be chosen in the range of from 30:1 to 10:1. This washing extract is then added to the above base mixture.

After the reaction mixture of step (iv) has been added, and optionally with the washing extract as described above, it is preferred to heat the thus obtained mixture to a temperature in the range of from 70 to 90°C, preferably from 80 to 85°C for a time period usually in the range of from 30 to 120 mins. Thereafter, generally the mixture is allowed to settle, usually for 10 to 90 minutes at a temperature in the range of from 70 to 90°C, preferably from 75 to 85°C.

In an optional and preferred embodiment, the lower aqueous layer is removed and the base treatment is repeated. In a particular embodiment, the thus obtained organic layer is allowed to settle at an elevated temperature, preferably in the range of from 60 to 90°C, for a period of time in the range of from 30 minutes to 4 hours.

Therefore, a preferred embodiment of the present invention concerns in step (v) after treating the organic phase with an aqueous base, the additional steps of removing the basic aqueous phase, and treating the remaining organic phase as econd time with an aqueous base before step (vi).

I.e. in this specific embodiment the following steps are carried out:
(i) reacting 4-methoxy-2-methyldiphenylamine with 2-(4'-di-n-butylamino-2'-hydroxybenzoyl)benzoic acid in fuming sulphuric acid (oleum) or a mixture thereof with concentrated sulphuric acid below 30°C
(ii) adding water to the reaction mass
(iii) adding a non-polar solvent to the diluted reaction mass
(iv) separating the organic phase
(v-a) adding an aqueous base to the organic phase and then heating at a temperature of 50° to 90°C,
(v-b) removal of the basic aqueous phase, and treating the organic phase a second time with an aqueous base to the remaining organic phase,
(vi) isolating 3-dibutylamino-6-methyl-7-anilinofluoran.

In step (vi) of the present invention the product is isolated. In principle the workup can be carried out straightforwardly by well-known methods, e.g. by separating the aqueous from the organic phase, then removing the organic solvent(s), and optionally washing and/or recrystallization of the product.

In a preferred embodiment the non-polar solvent is distilled off, preferably in an atmosphere under reduced pressure, and the product is obtained by filtration with optional washing and re-crystallization steps.

In an even more preferred embodiment, before the distillation is carried out an aqueous base is added to the reaction mass.

Typically, the same aqueous base is used as in the previous step(s). Generally, the dry weight ratio of 2-(4'-di-n-butylamino-2'-hydroxybenzoyl)benzoic acid to the base in the range of from 50:1 to 100:1. And usually, the aqueous base is used in a concentration in the range of from 10 to 50% b.w.

After removal of the organic solvent(s), usually the hot aqueous slurry with the product is transferred to a filter equipped with an agitator. When the transfer is complete, the agitation is stopped and the slurry is filtered. Optionally, washing with a polar solvent such as methanol can be carried out if desired. Preferably, after the filtration is complete, the filter residue is dried with agitation on the preferably heated filter.

A further embodiment of the present invention concerns a heat or pressure sensitive recording material comprising 3-dibutylamino-6-methyl-7-anilinofluoran.

The heat sensitive recording material of the invention can be prepared according to conventional methods e.g. described in EP 1,140,515, which is incorporated as reference. Further details are given in the examples below. For example, at least 3-dibutylamino-6-methyl-7-anilinofluoran as colour forming compound, at least one developer such as N-(p-toluenesulphonyl)-N'-(3-p-toluenesulphonyloxyphenyl) urea (sold as Ciba®Pergafast®201) and, if desired, at least one sensitiser are pulverised separately in water or a suitable dispersing medium, such as aqueous polyvinyl alcohol, to form an aqueous or other dispersion. If desired a stabiliser is treated in the same manner. The fine particle dispersions thus obtained are combined and then mixed with conventional amounts of binder, filler and lubricant.

Further representative developers used for the heat or pressure sensitive recording material include 2,2-bis(4-hydroxyphenyl)propane (bis phenol A), 2,2-bis(4'-hydroxyphenyl)-4-methylpentane, benzyl-4-hydroxybenzoate, 4,4'-dihydroxydiphenylsulfone, 2,4'-dihydroxydiphenylsulfone, 4-hydroxy-4'-isopropoxydiphenylsulfone (as sold for example under the tradename D8 by Nippon Soda), 2,2'-diallyl-4,4'-sulfonyldiphenol (as sold for example under the tradename TG-SA by Nippon Kayaku), phenol, 4,4'-sulfonylbis-polymer with 1,1'-oxobis(2-chloroethane) (as sold for example under the tradename D90 by Nippon Soda), 4-[(4-(1-methylethoxy)phenyl)sulfonyl]-phenol and carbamic acid, N,N'-[sulfonylbis[4,1-phenyleneiminocarbonylimino(methylphenylene)]]bis-, C,C'-diphenyl ester (as sold by Asahi Denka Kogyo under the tradename UU), 4,4'-bis(*p*-toluenesulphonylaminocarbonylamino)diphenylmethane) (as sold for example under the tradename B-TUM), zinc bis[(4-n-octyloxycarbonylamino)salicylate] dihydrate (as sold for example under the tradename SZ-110 by Mitsui Chemicals), 4-hydroxybenzoate derivative of a polypentaerythritol compound with CAS number 378244-93-0 as sold for example by Asahi Denka Kogyo under the tradename K5, and mixtures thereof.

Representative sensitisers include stearamide, amide waxes, p-benzylbiphenyl, 1,2-diphenoxyethane, 1,2-bis(3-methylphenoxy)ethane, benzyl-2-naphthyl ether, dibenzyl oxalate and di-(4-methylbenzyl) oxalate.

Representative binders used for the heat sensitive recording material include polyvinyl alcohol (fully and partially hydrolysed), carboxy, amide, sulfonic, silanol and butyral modified polyvinyl alcohols, derivatives of cellulose such as hydroxyethyl cellulose, methyl cellulose, ethyl cellulose, carboxymethyl cellulose and acetyl cellulose, copolymer of styrene-maleic anhydride, copolymer of styrene-butadiene, polyvinyl chloride, polyvinyl acetate, polyacrylamide, polyamide resin and mixtures thereof.

Exemplary fillers which can be used include ground and precipitated calcium carbonate, kaolin, calcined kaolin, aluminium hydroxide, talc, titanium dioxide, zinc oxide, amorphous silica, polystyrene resin, urea-formaldehyde resin, hollow plastic pigment and mixtures thereof.

Representative lubricants for use in heat sensitive recording materials include dispersions or emulsions of stearamide, methylene bisstearamide, polyethylene, carnauba wax, paraffin wax, zinc stearate or calcium stearate and mixtures thereof.

Other additives can also be employed, if necessary. Such additives are for example fluorescent whitening agents and ultraviolet absorbers.

The coating composition so obtained can be applied to a suitable substrate such as paper, plastic sheet and resin coated paper, and used as the heat sensitive recording material. The system of the invention can be employed for other end use applications using colour forming materials, for example, a temperature indicating material.

The quantity of the coating is usually in the range of 1 to 10 g/m², most often in the range 2 to 5 g/m²_{.}

The recording material containing such a thermosensitive colouring layer can in addition contain a protective layer and, if desired, an undercoat layer. The undercoat layer may be interposed between the substrate and the thermosensitive colouring layer.
The protective layer usually comprises a water-soluble resin in order to protect the thermosensitive colouring layer. If desired, the protective layer may contain water-soluble resins in combination with water-insoluble resins.

As such resins conventional resins can be employed. Specific examples are:
polyvinyl alcohol; starch and starch derivatives; cellulose derivatives such as methoxycellulose, hydroxyethylcellulose, carboxymethylcellulose, methylcellulose and ethylcellulose; sodium polyacrylate; polyvinyl pyrrolidone; polyacrylamide/acrylic acid ester copolymers; acrylamide/acrylic acid ester/methacrylic acid copolymers; alkali metal salts of styrene/maleic anhydride copolymers; alkali metal salts of isobutylene/maleic anhydride copolymers; polyacrylamide; sodium alginate; gelatin; casein; water-soluble polyesters and carboxyl-group-modified polyvinyl alcohols.

The protective layer may also contain a water-resisting agent such as a polyamide resin, polyamide-epichlorohydrin resin, melamine resin, formaldehyde, glyoxal or chromium alum.

Furthermore, the protective layer may contain fillers, such as finely-divided inorganic powders, e.g. of calcium carbonate, amorphous silica, zinc oxide, titanium oxide, aluminium hydroxide, zinc hydroxide, barium sulphate, clay, talc, surface-treated calcium or silica, or a finely-divided organic powder of, e.g., a urea-formaldehyde resin, a styrene/methacrylic acid copolymer or polystyrene.

The undercoat layer usually contains as its main components a binder resin and a filler.

Specific examples of binder resins for use in the undercoat layer are:
polyvinyl alcohol; starch and starch derivatives; cellulose derivatives such as methoxycellulose, hydroxyethylcellulose, carboxymethylcellulose, methylcellulose and ethylcellulose; sodium polyacrylate; polyvinyl pyrrolidone; polyacrylamide/acrylic acid ester copolymers; acrylamide/acrylic acid ester/methacrylic acid copolymers; alkali metal salts of styrene/maleic anhydride copolymers; alkali metal salts of isobutylene/maleic anhydride copolymers; polyacrylamide; sodium alginate; gelatin; casein; water-soluble polymers such as water-soluble polyesters and carboxyl-group-modified polyvinyl alcohols; polyvinyl acetate; polyurethanes; styrene/butadiene copolymers; polyacrylic acid; polyacrylic acid esters; vinyl chloride/vinyl acetate copolymers; polybutylmethacrylate; ethylen/vinylacetate copolymers and styrene/butadiene acrylic derivative copolymers.

Specific examples of fillers for use in the undercoat layer are:
finely-divided inorganic powders, e.g. of calcium carbonate, silica, zinc oxide, titanium oxide, aluminium hydroxide, zinc hydroxide, barium sulphate, clay, talc, surface-treated calcium, silica or calcined clay (eg Ansilex,Engelhard Corp.), and finely-divided organic powders of, e.g., urea-formaldehyde resins, styrene/methacrylic acid copolymers, polystyrene and hollow plastic pigment.

In addition, the undercoat layer may contain a water-resisting agent. Examples of such agents are given above.

The inventive process has the advantage that a white 3-dibutylamino-6-methyl-7-anilinofluoran is obtained, that the used sulfuric acid can be recovered easily, and that fewer by-products are observed. Moreover, beige and pink colored 3-dibutylamino-6-methyl-7-anilinofluoran can be avoided, and thus the background whiteness of the recording material can be improved.

### Examples

Example 1: A glass vessel was charged with stirring 98% sulphuric acid (6.000 kg) followed by oleum containing 20% SO₃ (1.953 kg), followed by 98% sulphuric acid (2.060 kg). The acid mixture was cooled to 17°C and 2'-carboxy-4-dibutylamino-2-hydroxybenzophenone (3.106 kg) was added in portions over 6 hours while maintaining the temperature below 20°C. The mixture was then cooled to 15°C over 45 mins and 4-methoxy-2-methyldiphenylamine (1.848 kg) was added in portions over 5 hours maintaining the temperature below 20°C. Water (1.458 kg, having a temperature of 20°C) was then added over 3 hours to the externally cooled reaction mixture, maintaining a temperature below 47°C. Then further water (4.572 kg, 20°C) was added over 30 mins, maintaining the reaction mass temperature below 70°C. Whilst maintaining a temperature in the range of 65 to 70°C, toluene (4.848 kg) was added. Then the stirring was stopped and the mixture allowed to settle for 165 mins at 65°C.

Thereafter lower acid layer was then separated and afterwards methanol (0.450 kg) was added to the toluene layer which was then stirred at 65±2°C for 30 mins.
A separate vessel was charged with hot water (6.255 kg) followed by 32% by weight sodium hydroxide solution (11.953 kg), followed by toluene (9.043 kg). Whilst maintaining this mixture at 75±2°C, the toluene / methanol / phthalide mixture from the previous step was then pumped into the vessel over a period of 150 mins. Methanol (0.250 kg, 60°C) was used to wash out the phthalide mixture from the vessel and the washings were transferred to the toluene / aqueous base mixture. The thus obtained mixture was then heated at 82±2°C for 90 mins. The stirring was stopped and the mixture allowed to settle for 30 mins at 80±2°C. Then the lower aqueous layer was removed and the toluene layer stirred whilst water (3.776 kg, 60°C) and 32% b.w. sodium hydroxide solution (0.294 kg) were added. The temperature was adjusted to 80±2°C and held for 15 mins. The stirring was stopped and the mixture allowed settling for 2 hours at 80±2°C. The lower aqueous layer was separated.

A separate vessel equipped for distillation and a separator to return water was charged with water (0.953 kg, 60°C) and 32% b.w. sodium hydroxide solution (0.147 kg). The toluene solution from the previous step was then pumped into the vessel. Separately, toluene (0.400 kg, 60°C) and water (1.000 kg, 60°C) were used to wash out the vessel in the previous step and the washings transferred to the aqueous base mixture. The mixture was stirred and the temperature adjusted to 75°C.

The pressure in the vessel was reduced to 300 mbar and toluene removal by distillation was commenced. When approximately one third of the reaction mixture weight was removed, an equivalent amount of water was added to the vessel to maintain an approximate constant weight. When the temperature reaches 65°C, the pressure was increased to 400 mbar and distillation was continued. The toluene removal was complete when the temperature reaches 75°C, whereupon the vacuum was released.

The hot aqueous slurry of product was then transferred to a filter equipped with an agitator. When transfer was complete, the agitation is stopped and the slurry is filtered. Hot methanol (4.580 kg) was used to wash out the vessel in the previous step and the washings were transferred to the filter. The resulting slurry was then heated at 63°C with agitation on the filter for 30 mins. The agitator was then stopped and the slurry filtered. Methanol (3.500 kg) was added to the filter residue with agitation before the slurry was filtered again. This procedure was repeated using a further 3.500 kg methanol as a final wash. The slurry was allowed to sit on the filter for a 25 mins before the final filtration. After the filtration was complete, the filter residue was dried with agitation on the heated filter. 3-(dibutylamino)-6-methyl-7-anilinofluoran was obtained as a white solid, melting point 184°C. Yield 4.000 kg (89.6%).

### Example 2: Preparation of a heat sensitive recording material

Mean particle sizes were determined using a Coulter LS 230 laser diffraction particle size analyzer.

### Preparation of Dispersion A (Colour Former)

| | parts |
|---|---|
| 3-di-butylamino-6-methyl-7-anilinofluoran (product of Example 1) | 10.0 |
| 10% PVA 203 ⁽¹⁾ solution | 20.0 |
| wetting agent (Surfynol 104 ⁽²⁾ | 0.1 |
| Water | 9.9 |

| | |
|---|---|
| ¹ partially hydrolysed poly vinylalcohol from Kuraray Co. Ltd. ² 2,4,7,9-tetramethyldec-5-yne-4,7-diol manufactured by Air Products & Chemicals Inc. | |

The mixture of the above components was milled in a horizontal bead mill to a mean particle size of 1.0 µm.

### Preparation of Dispersion B (Colour Developer)

| | parts |
|---|---|
| Bisphenol A | 20.0 |
| 10% PVA 203 solution | 13.3 |
| 45% sodium naphthalene sulfonate, polymer with formaldehyde solution ⁽³⁾ | 0.5 |
| water | 46.2 |

| | |
|---|---|
| ³ commercially available e.g. as Tamol NN 9401 manufactured from BASF | |

The mixture of the above components was milled in a horizontal bead mill to a mean particle size of 1.0 µm.

### Preparation of Dispersion C (Sensitiser)

| | Parts |
|---|---|
| 1,2-Di(3-methylphenoxy)ethane ⁽⁴⁾ | 20.0 |
| 10% PVA 203 solution | 6.7 |
| 45% sodium naphthalene sulfonate, polymer with formaldehyde solution | 0.5 |
| water | 52.8 |

| | |
|---|---|
| ⁴ commercially available e.g. as Ciba®Pergaspeed™307 | |

The mixture of the above components was milled in a horizontal bead mill to a mean particle size of 1.0 µm.

### Preparation of Dispersion D (Pigment)

| | parts |
|---|---|
| precipitated calcium carbonate ⁽⁵⁾ | 40.0 |
| 40% sodium polyacrylate solution | 0.4 |
| water | 59.6 |

| | |
|---|---|
| ⁵ commercially available e.g. as Socal P3 manufactured by Solvay S.A. (i.e. an uncoated calcite, crystal class: scalenohedral, cigar like crystal shape, mean particle size: 200 to 300 nm, specific surface area: 6 to 10 m²/g, brightness: 97 to 98%, flow point: 15 to 45 cm³/15 g) | |

The mixture of the above components was milled in a horizontal bead mill to a mean particle size of 1.0 µm.

80 parts of Dispersion A, 160 parts of Dispersion B, 160 parts of Dispersion C, 75 parts of Dispersion D, 21.7 parts of a 30% zinc stearate dispersion (Hidorin Z-7 manufactured by Chukyo Europe), 48.4 parts of 20% PVA 203 solution and 2.2 parts of a fluorescent whitening agent (Ciba®Tinopal® ABP-Z Liquid)were mixed together with stirring.

The coating composition thus obtained was applied with a dry coatweight of 4.7 g/m² to a base paper (pre-coated with calcined clay,) weighing 50 g/m². After drying, the resulting heat sensitive paper was calendered to 400 Bekk seconds smoothness.

### Example 3: Evaluation of Heat Sensitive Recording Materials

The heat sensitive recording material prepared above was evaluated as described below:

### Thermal Printing

Using a Thermal Tester (Atlantek Model 200 manufactured by Atlantek Inc.), the heat sensitive recording material was printed with an applied energy of 0.50 mJ/dot.

### Optical Density Measurements

The optical densities of the imaged and background portions of the heat sensitive material were measured with a Gretag SPM50 spectrophotometer.

### Static Sensitivity

The heat sensitive recording material was exposed to a range of temperatures for a fixed period of 5 seconds. Twelve separately heated blocks at 60, 65, 70, 75, 80, 85, 90, 95, 100, 110, 120 and 130°C were applied to the paper to produce an image. The optical density of each image was measured.

| Temp (°C) | 60 | 65 | 70 | 75 | 80 | 85 | 90 | 95 | 100 | 110 | 120 | 130 |
|---|---|---|---|---|---|---|---|---|---|---|---|---|
| O.D. | 0.02 | 0.02 | 0.03 | 0.06 | 0.14 | 0.31 | 0.76 | 0.83 | 0.85 | 0.94 | 0.92 | 0.91 |

### Dynamic Sensitivity

Ten individual 1cm x 1cm squares were printed with increased amounts of energy using an Atlantek thermal response tester, model 200. The optical density of each image was measured.

| Energy(mJ/dot) | 0.05 | 0.10 | 0.15 | 0.20 | 0.25 | 0.30 | 0.30 | 0.40 | 0.45 | 0.50 |
|---|---|---|---|---|---|---|---|---|---|---|
| O.D. | 0.03 | 0.06 | 0.24 | 0.58 | 1.02 | 1.22 | 1.29 | 1.30 | 1.26 | 1.25 |

### Heat / Humidity Resistance

After printing, the heat sensitive recording material was stored for 24 hours in an oven maintained at 40°C and 90% relative humidity. The optical densities of the imaged and background portions were then measured.

| | image | background |
|---|---|---|
| initial | 1.25 | 0.03 |
| after 24 hr/ 40°C/ 90% RH | 1.18 | 0.03 |

### Example 4: Preparation of a pressure sensitive recording material

A 4% b.w. solution of the product of Example 1 was dissolved in KMC oil (mixture of diisopropylnaphthalenes manufactured by RKS GmbH, viscosity: 16mPas (20°C), a solvent used for carbonless copy paper) and encapsulated using a melamine-formaldehyde wall material as described in example 1 of WO03/035245 A1 to produce a 35% active content microcapsule dispersion.

A coating composition was prepared as follows :

| | Parts by weight |
|---|---|
| microcapsule dispersion | 73.1 |
| Arbocel® BE 600 / 30⁽⁶⁾ | 10.2 |
| binder (DL 950⁽⁷⁾) | 10.2 |
| water | 156.5 |

| | |
|---|---|
| ⁶ a cellulose fibre spacer supplied by Rettenmaier & Söhne GmbH. ⁷ a 50% aquesous dispersion of a carboxylated styrene-butadiene copolymer supplied by Dow Chemical Company Ltd. | |

The above composition was applied at a coat weight of 5g /m² to a base paper. The resulting CB (Coated Back) sheet was placed coated side down on top of sheets of commercial clay, CF paper (Coated Front) coated side up. A CB to CF print was then made using a dot matrix printer. The optical density and shade of the image produced on the CF sheet were measured after 2 minutes, 1 hour and 24 hours, respectively, with a Gretag SPM 50 spectrophotometer.

### Optical Density

| 2 mins | 1 hour | 24 hours |
|---|---|---|
| 0.45 | 0.47 | 0.48 |

| 2 mins | | | 1 hour | | | 24 hours | | |
|---|---|---|---|---|---|---|---|---|
| **L*** | **a*** | **b*** | **L*** | **a*** | **b*** | **L*** | **a*** | **b*** |
| 70.6 | 0.3 | -3.5 | 69.8 | -0.5 | -3.4 | 69.0 | -0.1 | -3.7 |

### Example 5: A 3% by weight solution of the colour former is prepared in a mixture of

KMC / Exxsol® D-100 (70/30 w/w). The absorption of the solution is measured using a Hitachi U-2800A UV/VIS spectrophotometer. The results are shown in Table 1.

**Table 1**

| | Absorption | | |
|---|---|---|---|
| wavelength (nm) | Product of Example 1 | Comparative Example 1^{(*)} | Comparative Example 2^{(**)} |
| 400 | 0.06 | 0.40 | 0.19 |
| 425 | 0.06 | 0.35 | 0.17 |
| 450 | 0.06 | 0.25 | 0.15 |
| 475 | 0.06 | 0.23 | 0.14 |
| 500 | 0.05 | 0.22 | 0.09 |
| 525 | 0.03 | 0.21 | 0.09 |
| 550 | 0.03 | 0.20 | 0.06 |
| 575 | 0.03 | 0.18 | 0.05 |
| 600 | 0.03 | 0.17 | 0.04 |
| 625 | 0.03 | 0.15 | 0.03 |
| 650 | 0.03 | 0.14 | 0.03 |
| 675 | 0.03 | 0.12 | 0.03 |
| 700 | 0.03 | 0.11 | 0.03 |

| | | | |
|---|---|---|---|
| (*) light beige product, melting point 183.4 -184.3°C corresponding to Example 11 of US 5,166,350 (**) light pink product, melting point 183.6 -184.4°C corresponding to Example 12 of US 5,166,350 | | | |

The absorption of the solution in the 400-700 nm range provides a direct measure of the coloured impurities present in the sample. The results clearly demonstrate the superior quality of 3-dibutylamino-6-methyl-7-anilinofluoran produced by the process of the invention.

## Claims

1. A process for the preparation of 3-dibutylamino-6-methyl-7-anilinofluoran which process comprises reacting 4-methoxy-2-methyldiphenylamine with 2-(4'-di-n-butylamino-2'-hydroxybenzoyl)benzoic acid in fuming sulphuric acid or a mixture thereof with sulphuric acid, treating the reaction mass with water, a non-polar solvent and a base, and isolating the final product, **characterized in** carrying out the following steps:
(i) carrying out the reaction between 4-methoxy-2-methyldiphenylamine and 2-(4'-di-n-butylamino-2'-hydroxybenzoyl)benzoic acid in fuming sulphuric acid or a mixture thereof with sulphuric acid at a temperature below 30°C,
(ii) then adding water to the reaction mass,
(iii) afterwards adding a non-polar solvent to the diluted reaction mass,
(iv) followed by separating the obtained organic phase,
(v) thereafter treating the organic phase with an aqueous base, and then heating at a temperature of 50° to 90°C, and finally
(vi) isolating 3-dibutylamino-6-methyl-7-anilinofluoran.

2. A process according to claim 1, in which in step (v) after treating the organic phase with an aqueous base, the additional steps of removing the basic aqueous phase, and treating the remaining organic phase a second time with an aqueous base before step (vi) are carried out.

3. A process according to claim 1 in which the reaction of 4-methoxy-2-methyldiphenylamine with 2-(4'-di-n-butylamino-2'-hydroxybenzoyl)benzoic acid in concentrated or fuming sulphuric acid or a mixture thereof is carried out at a temperature of 10° to 20°C

4. A process according to claim 1 in which the addition of water to the reaction mass is carried out at 30 to 70°C

5. A process according to claim 1 in which the non-polar solvent is added to the diluted reaction mass at 60 to 80°C

6. A process according to claim 5 in which the non-polar solvent is added to the diluted reaction mass at 65 to 70°C

7. A process according to claim 1 in which the aqueous base is sodium hydroxide or potassium hydroxide

8. A process according to claim 1 in which the aqueous base and organic phase are heated together at a temperature of 70 to 85°C.

9. A process according to claim 1 in which the organic solvent is toluene or xylene.

10. A heat or pressure sensitive recording material comprising 3-dibutylamino-6-methyl-7-anilinofluoran prepared according to claims 1 to 9.
